# EUROPEAN PATENT APPLICATION

(11) **EP 0 880 939 A1**
(43) Date of publication of application: **02.12.1998**
(21) Application number: 98304173.2
(22) Date of filing: 27.05.1998
(51) Int. Cl.: A61B 17/34

(54) **Ultrasonic trocar assembly**

(30) Priority: 27.05.1997 US 863799
(71) Applicant: ETHICON ENDO-SURGERY, INC., Cincinnati, Ohio 45242 (US)
(72) Inventor: Wampler, Scott D., Loveland, Ohio 45140 (US); Privitera, Salvatore, West Chester, Ohio 45069 (US)
(74) Representative: Mercer, Christopher Paul

(57) **Abstract**

An ultrasonic trocar assembly includes an ultrasonic obturator and associated trocar cannula which can be used together for effecting placement of the trocar cannula in a patient for performing endoscopic surgical procedures. The ultrasonic obturator includes an elongated waveguide which transmits ultrasonic energy to a distal end effector of the waveguide, with the end effector being positionable against the wall of a body cavity of the patient for creating a penetration opening. The ultrasonic obturator includes a non-vibratory, sheath-like interface member surrounding and diverging outwardly from the end effector of the waveguide for dilating and enlarging the initially formed penetration opening, thereby minimizing patient trauma and facilitating rapid patient recovery.

## Description

### Technical Field

The present invention relates generally to an ultrasonic trocar assembly for use in connection with endoscopic surgical procedures, and more particularly to a trocar assembly including an ultrasonic trocar obturator having a shield-like interface member at a distal end thereof for effecting dilation of a relatively small penetration opening formed ultrasonically by the obturator.

### Background Of The Invention

Minimally invasive endoscopic surgical procedures have become increasingly widespread in view of the reduced trauma for patients, and their highly cost-effective nature. For such procedures, a trocar assembly is typically employed for effecting penetration of the wall of a body cavity of the patient. A typical trocar assembly includes an outer, tubular cannula, and an inner tissue. piercing obturator. For use, the trocar assembly is positioned so that the obturator can be advanced to pierce the wall of the body cavity, and the cannula thereafter inserted through the opening. The obturator is then removed from within the cannula, with the cannula thereafter providing access for insertion of surgical instruments and other devices related to the surgical procedure to be performed.

Use of ultrasonic surgical instruments is also becoming increasingly widespread by virtue of the unique performance characteristics of such devices. Depending upon specific instrument configuration and operational parameters, such devices can provide substantially simultaneous cutting of tissue and hemostasis, again desirably minimizing patient trauma.

U.S. Patent No. 5,449,370, hereby incorporated by reference, discloses an ultrasonic trocar assembly including an ultrasonic obturator configured to effect ultrasonic penetration of the wall of a body cavity for subsequent introduction of a tubular cannula of the trocar assembly. The obturator of the trocar assembly is provided with a blunt or rounded tip for minimizing patient trauma attendant to penetration of the body cavity.

The present invention is directed to an ultrasonic trocar assembly configured to minimize patient trauma, thus facilitating versatile application and promoting rapid patient recovery.

### Summary Of The Invention

An ultrasonic trocar assembly embodying the principles of the present invention includes an ultrasonic trocar obturator configured for use with an associated tubular trocar cannula. The obturator includes an end effector portion which is ultrasonically energized for effecting penetration of the wall of a body cavity of a patient. Additionally, the obturator includes a non-vibratory sheath-like interface member which surrounds and diverges outwardly of the end effector. This arrangement permits ultrasonic penetration of the body cavity, followed by dilation of the penetration opening by the non-vibratory interface member.

In accordance with the illustrated embodiment, the ultrasonic obturator of the trocar assembly includes a housing having an ultrasonic transducer assembly positioned therein. A generally elongated ultrasonic waveguide extends from the housing, and includes a proximal end operatively coupled to the transducer assembly for transmission of ultrasonic energy through the waveguide. The waveguide includes an end effector at the distal end thereof, preferably configured as a generally rounded blunt or non-sharpened tip portion.

In accordance with the present invention, an interface member surrounds and diverges outwardly of the end effector of the waveguide. The interface member preferably includes a distal portion spaced from the free end of the end effector to thereby define an exposed end portion thereof. The interface member has an outwardly, rearwardly diverging configuration, which may be generally frusto-conical or curved, and which is configured to effect dilation of the penetration opening formed by the end effector of the associated waveguide. It is contemplated that the interface member taper outwardly at an angle of about 10° to about 60° from the centerline of the waveguide of the obturator.

In use, the ultrasonic obturator and trocar cannula of the trocar assembly are coaxially positioned in generally telescopic relationship with each other, with the obturator slidably disposed within the tubular cannula. The obturator is positioned in contact with the wall of the body cavity of the patient, with application of ultrasonic energy through the waveguide of the obturator creating a penetration opening in the wall. The penetration opening is subsequently dilated by distally advancing the obturator so that the sheath-like interface members surrounding the end effector of the waveguide acts to enlarge the initially formed opening. The trocar cannula of the assembly can thereafter be inserted into the dilated and enlarged penetration opening, with removal of the obturator from within the cannula thereafter permitting performance of the desired endoscopic surgical procedure.

Other features and advantages of the present invention will become readily apparent from the following detailed description, the accompanying drawings, and the appended claims.

### Brief Description Of The Drawings

FIGURE 1 is a diagrammatic view of an ultrasonic surgical system which can be modified in accordance with the present invention to provide the present ultrasonic trocar assembly;
FIGURE 2 is an exploded perspective view of the ultrasonic trocar assembly embodying the principles of the present invention;
FIGURE 3 is an exploded perspective view of an alternate embodiment of the present ultrasonic trocar assembly;
FIGURE 4 is a partial cross-sectional view of an ultrasonic trocar assembly embodying the principles of the present invention in accordance with the embodiment of FIGURE 2;
FIGURE 5 is a fragmentary partial cross-sectional view of a distal end portion of the ultrasonic obturator of the present trocar assembly in accordance with the embodiment of FIGURE 2; and
FIGURE 6 is a view similar to FIGURE 5 further illustrating the alternate embodiment of the present ultrasonic trocar assembly shown in FIGURE 3.

### Detailed Description

While the present invention is susceptible of embodiment in various forms, there is shown in the drawings and will hereinafter be described a presently preferred embodiment, with the understanding that the present disclosure is to be considered as an exemplification of the invention, and is not intended to limit the invention to the specific embodiment illustrated.

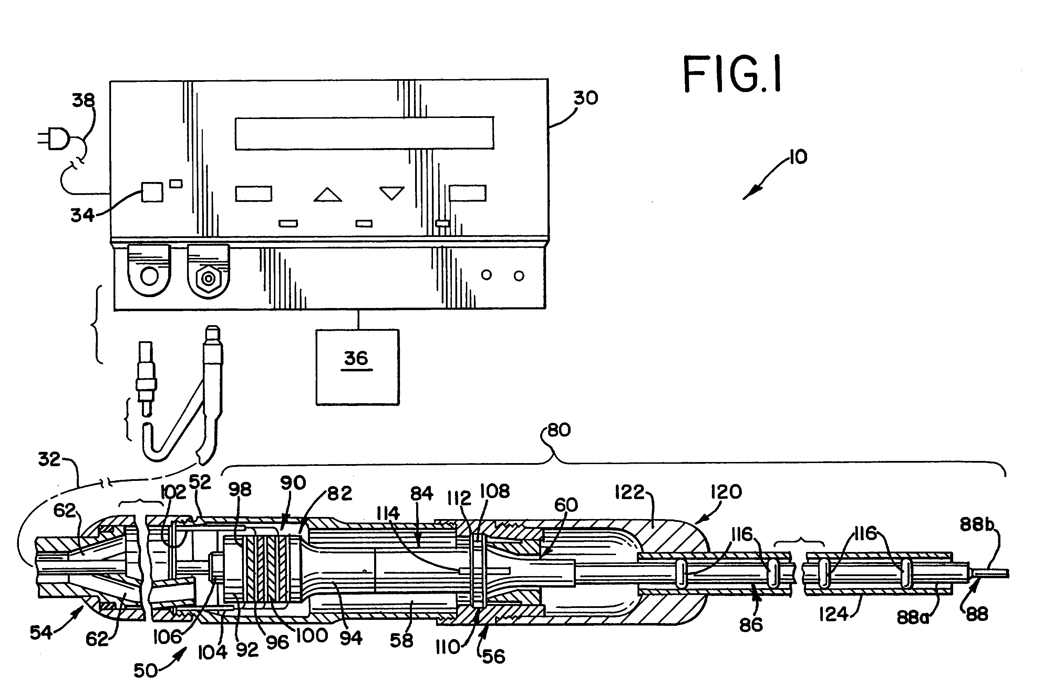

With reference to FIGURE 1, therein is illustrated an ultrasonic surgical system 10 configured in accordance with the present invention. Ultrasonic surgical 10 is illustrated generally in the form of an ultrasonic scalpel, but it will be understood that the present ultrasonic trocar assembly of the present invention is configured, in many respects, in accordance with ultrasonic surgical system 10. Thus, for purposes of the present disclosure, the surgical system 10 will be described in detail, with the understanding that the trocar assembly of the present invention is configured in accordance with the illustrated system 10, except as otherwise noted. The present ultrasonic trocar assembly differs from the surgical system 10 illustrated in FIGURE 1 in that the present trocar assembly includes an ultrasonic obturator useable in conjunction with a tubular trocar cannula. While the ultrasonic obturator of the present invention is configured and functions similarly to the illustrated instrument of ultrasonic surgical system 10, specific features of the present obturator, as will be further described, are particularly configured for cooperation with a trocar cannula for preparation of a patient for an endoscopic surgical procedure.

Referring to FIGURE 1, a presently preferred embodiment of the surgical system 10 is illustrated. The surgical system 10 generally includes a generator 30, a handpiece assembly 50, and an acoustic or transmission assembly 80. The generator 30 sends an electrical signal through a cable 32 at a selected amplitude, frequency, and phase determined by a control system of the generator 30. As will be further described, the signal causes one or more piezoelectric elements of the acoustic assembly 80 to expand and contract, thereby converting the electrical energy into mechanical motion. The mechanical motion results in longitudinal waves of ultrasonic energy that propagate through the acoustic assembly 80 in an acoustic standing wave to vibrate the acoustic assembly 80 at a selected frequency and amplitude. An end effector 88 at the distal end of the acoustic assembly 80 is placed in contact with tissue of the patient to transfer the ultrasonic energy to the tissue. The cells of the tissue in contact with the end effector 88 of the acoustic assembly 80 will move with the end effector 88 and vibrate.

As the end effector 88 couples with the tissue, thermal energy or heat is generated as a result of internal cellular friction within the tissue. The heat is sufficient to break protein hydrogen bonds, causing the highly structured protein (i.e., collagen and muscle protein) to denature (i.e., become less organized). As the proteins are denatured, a sticky coagulum forms to seal or coagulate small blood vessels when the coagulum is below 100°C. Deep coagulation of larger blood vessels results when the effect is prolonged.

The transfer of the ultrasonic energy to the tissue causes other effects including mechanical tearing, cutting, cavitation cell disruption, and emulsification. The amount of cutting as well as the degree of coagulation obtained varies with the vibrational amplitude of the end effector 88, the amount of pressure applied by the user, and the sharpness of the end effector 88. The end effector 88 of the acoustic assembly 80 in the surgical system 10 tends to focus the vibrational energy of the system 10 onto tissue in contact with the end effector 88, intensifying and localizing thermal and mechanical energy delivery.

As illustrated in FIGURE 1, the generator 30 includes a control system integral to the generator 30, a power switch 34, and a triggering mechanism 36. The power switch 34 controls the electrical power to the generator 30, and when activated by the triggering mechanism 36, the generator 30 provides energy to drive the acoustic assembly 80 of the surgical system 10 at a predetermined frequency and to drive the end effector 88 at a predetermined vibrational amplitude level. The generator 30 may drive or excite the acoustic assembly 80 at any suitable resonant frequency of the acoustic assembly 80.

When the generator 30 is activated via the triggering mechanism 36, electrical energy is continuously applied by the generator 30 to a transducer assembly 82 of the acoustic assembly 80. A phase locked loop in the control system of the generator 30 monitors feedback from the acoustic assembly 80. The phase lock loop adjusts the frequency of the electrical energy sent by the generator 30 to match a preselected harmonic frequency of the acoustic assembly 80. In addition, a second feedback loop in the control system maintains the electrical current supplied to the acoustic assembly 80 at a preselected constant level in order to achieve substantially constant vibrational amplitude at the end effector 88 of the acoustic assembly 80. The electrical signal supplied to the acoustic assembly 80 will cause the distal end to vibrate longitudinally in the range of, for example, approximately 20 kHz to 100 kHz, and preferably in the range of about 54 kHz to 56 kHz, and most preferably at about 55.5 kHz. The amplitude of the acoustic vibrations at the end effector 88 may be controlled by, for example, controlling the amplitude of the electrical signal applied to the transduction portion 90 of the acoustic assembly 80 by the generator 30.

As noted above, the triggering mechanism 36 of the generator 30 allows a user to activate the generator 30 so that electrical energy may be continuously supplied to the acoustic assembly 80. In one embodiment, the triggering mechanism 36 preferably comprises a foot activating switch that is detachably coupled or attached to the generator 30 by a cable or cord. In another embodiment, a hand switch may be incorporated in the handpiece assembly 50 to allow the generator 30 to be activated by a user.

The generator 30 also has a power line 38 for insertion in an electrosurgical unit or conventional electrical outlet. It is contemplated that the generator 30 may also be powered by a direct current (DC) source, such as a battery. The generator 30 may be any suitable generator, such as Model No. GENO1 available from Ethicon Endo-Surgery, Inc.

Referring still to FIGURE 1, the handpiece assembly 50 includes a multi-piece housing or outer casing 52 adapted to isolate the operator from the vibrations of the acoustic assembly 80. The housing 52 is preferably cylindrically shaped and is adapted to be held by a user in a conventional manner, but may be any suitable shape and size which allows it to be grasped by the user. While a multi-piece housing 52 is illustrated, the housing 52 may comprise a single or unitary component.

The housing 52 of the handpiece assembly 50 is preferably constructed from a durable plastic, such as Ultem®. It is also contemplated that the housing 52 may be made from a variety of materials including other polymers (i.e. high impact polystyrene or polypropylene). A suitable handpiece assembly 50 is Model No. HP050, available from Ethicon Endo-Surgery, Inc.

Referring still to FIGURE 1, the handpiece assembly 50 generally includes a proximal end 54, a distal end 56, and centrally disposed axial opening or cavity 58 extending longitudinally therein. The distal end 56 of the handpiece assembly 50 includes an opening 60 configured to allow the acoustic assembly 80 of the surgical system 10 to extend therethrough, and the proximal end 54 of the handpiece assembly 50 is coupled to the generator 30 by a cable 32. The cable 32 may include ducts or vents 62 to allow air to be introduced into the handpiece assembly 50 to cool the transducer assembly 82 of the acoustic assembly 80.

Referring still to FIGURE 1, the acoustic assembly 80 generally includes a transducer stack or assembly 82, a mounting device 84, a transmission rod or waveguide 86, and an end effector or applicator 88. The transducer assembly 82, mounting device 84, transmission rod 86, and the end effector 88 are preferably acoustically tuned such that the length of each component is an integral number of one-half system wavelengths (nλ/2) where the system wavelength λ is the wavelength of a preselected or operating longitudinal vibration frequency f of the acoustic assembly 80. It is also contemplated that the acoustic assembly 80 may incorporate any suitable arrangement of acoustic elements. For example, the acoustic assembly 80 may comprise a transducer assembly and an end effector (i.e., the acoustic assembly 80 may be configured without a mounting device and a transmission rod).

The transducer assembly 82 of the acoustic assembly 80 converts the electrical signal from the generator 30 into mechanical energy that results in longitudinal vibratory motion of the end effector 88 at ultrasonic frequencies. When the acoustic assembly 80 is energized, a vibratory motion standing wave is generated through the acoustic assembly 80. The amplitude of the vibratory motion at any point along the acoustic assembly 80 depends on the location along the acoustic assembly 80 at which the vibratory motion is measured. A minimum or zero crossing in the vibratory motion standing wave is generally referred to as a node N (i.e., where axial motion is usually minimal and radial motion is usually small), and an absolute value maximum or peak in the' standing wave is generally referred to as an antinode A. The distance between an antinode and its nearest node is one-quarter wavelength (λ/4).

As shown in FIGURE 1, the transducer assembly 82 of the acoustic assembly 80, which is known as a "Langevin stack", generally includes a transduction portion 90, a first resonator 92, and a second resonator 94. The transducer assembly 82 is preferably an integral number of one-half system wavelengths (nλ/2) in length. It is to be understood that the present invention may be alternatively configured to include a transducer assembly comprising a magnetostrictive, electromagnetic or electrostatic transducer.

The distal end of the first resonator 92 is connected to the proximal end of transduction section 90, and the proximal end of the second resonator 94 is connected to the distal end of transduction portion 90. The first and second resonators 92 and 94 are preferably fabricated from titanium, aluminum, steel, or any other suitable material. The first and second resonators 92 and 94 have a length determined by a number of variables, including the thickness of the transduction section 90, the density and modulus of elasticity of material used in the resonators 92 and 94, and the fundamental frequency of the transducer assembly 82. The second resonator 94 may be tapered inwardly from its proximal end to its distal end to amplify the ultrasonic vibration amplitude.

The transduction portion 90 of the transducer assembly 82 preferably comprises a piezoelectric section of alternating positive electrodes 96 and negative electrodes 98, with piezoelectric elements 100 alternating between the electrodes 96 and 98. The piezoelectric elements 100 may be fabricated from any suitable material, such as lead zirconate, lead titanate, or ceramic crystal material. Each of the positive electrodes 96, negative electrodes 98, and piezoelectric elements 100 may have a bore extending through the center. The positive and negative electrodes 96 and 98 are electrically coupled to a wires 102 and 104, respectfully. Wires 102 and 104 transmit electrical signal from the generator 30 to electrodes 96 and 98.

As shown in FIGURE 1, the piezoelectric elements 100 are held in compression between the first and second resonators 92 and 94 by a bolt 106. The bolt 106 preferably has a head, a shank, and a threaded distal end. The bolt 106 is inserted from the proximal end of the first resonator 92 through the bores of the first resonator 92, the electrodes 96 and 98 and piezoelectric elements 100. The threaded distal end of the bolt 106 is screwed into a threaded bore in the proximal end of second resonator 94.

The piezoelectric elements 100 are energized in response to the electrical signal supplied from the generator 30 to produce an acoustic standing wave in the acoustic assembly 80. The electrical signal causes disturbances in the piezoelectric elements 100 in the form of repeated small displacements resulting in large compression forces within the material. The repeated small displacements cause the piezoelectric elements 100 to expand and contract in a continuous manner along the axis of the voltage gradient, producing high frequency longitudinal waves of ultrasonic energy. The ultrasonic energy is transmitted through the acoustic assembly 80 to the end effector 88.

The mounting device 84 of the acoustic assembly 80 has a proximal end, a distal end, and may have a length substantially equal to an integral number of one-half system wavelengths. The proximal end of the mounting device 84 is preferably axially aligned and coupled to the distal end of the second resonator 94 by an internal threaded connection at or near an antinode. It is also contemplated that the mounting device 84 may be attached to the second resonator 94 by any suitable means, and that the second resonator 94 and mounting device 84 may be formed as a single or unitary component.

The mounting device 84 is coupled to the housing 52 of the handpiece assembly 50 near a node. (For purposes of this disclosure, the term "near" is defined as "exactly at" or "in close proximity to".) The mounting device 84 may also include an integral ring 108 disposed around its periphery. The integral ring 108 is preferably disposed in an annular groove 110 formed in the housing 52 of the handpiece assembly 50 to couple the mounting device 84 to the housing 58. A compliant member or material 112, such as a pair of silicone O-rings attached by stand-offs, may be placed between the annular groove 110 of the housing 52 and the integral ring 108 of the mounting device 86 to reduce or prevent ultrasonic vibration from being transmitted from the mounting device 84 to the housing 52.

The mounting device 84 may be secured in a predetermined axial position by a plurality of pins 114, preferably four. The pins 114 are disposed in a longitudinal direction 90 degrees apart from each other around the outer periphery of the mounting device 84. The pins 114 are coupled to the housing 52 of the handpiece assembly 50 and are disposed through notches in the integral ring 108 of the mounting device 84. The pins 114 are preferably fabricated from stainless steel.

The mounting device 84 is preferably configured to amplify the ultrasonic vibration amplitude that is transmitted through the acoustic assembly 80 to the distal end of the end effector 88. In one preferred embodiment, the mounting device 84 comprises a solid, tapered horn. As ultrasonic energy is transmitted through the mounting device 84, the velocity of the acoustic wave transmitted through the mounting device 84 is amplified. It is contemplated that the mounting device 84 may be any suitable shape, such as a stepped horn, a conical horn, an exponential horn, or the like.

The distal end of the mounting device 84 may be coupled to the proximal end of the transmission rod 86 by an internal threaded connection. It is contemplated that the transmission rod 86 be attached to the mounting device 84 by any suitable means. The mounting device 84 is preferably coupled to the transmission rod 86 near or at an antinode.

The transmission rod 86 may, for example, have a length substantially equal to an integer number of one-half system wavelengths (nλ/2). The transmission rod 86 is preferably fabricated from a solid core shaft constructed out of material which propagates ultrasonic energy efficiently, such as titanium alloy (i.e., Ti-6Al-4V) or an aluminum alloy. The transmission rod 86 may be fabricated from other suitable materials. The transmission rod 86 may also amplify the mechanical vibrations transmitted through the transmission rod 86 to be end effector 88 as is well known in the art.

As illustrated in FIGURE 1, the transmission rod 86 includes stabilizing silicone rings or compliant supports 116 positioned at a plurality of nodes. The silicone rings 116 dampen undesirable vibration and isolate the ultrasonic energy from a tubular member of a removable sheath 120 assuring the flow of ultrasonic energy in a longitudinal direction to the distal end of the end effector 88 with maximum efficiency.

As shown in FIGURE 1, the removable sheath 120 is coupled to the distal end 56 of the handpiece assembly 50. The sheath 120 generally includes an adapter or nose cone 122 attached to an elongated tubular member 124 having an opening extending longitudinally therethrough. The sheath 120 may be threaded or snapped onto the distal end of the housing 52. The transmission rod 86 of the acoustic assembly 80 extends through the opening of the tubular member 124 and the silicone rings 116 support the transmission rod 86 therein. The adapter 122 of the sheath 120 is preferably constructed from Ultem® and the tubular member 124 is fabricated from stainless steel. Alternatively, the transmission rod 86 may have polymerid material that surrounds the transmission rod 86 to isolate it from outside contact.

The distal end of the transmission rod 86 is coupled to the proximal end of the end effector 88 by an internal threaded connection, preferably at or near an antinode. It is contemplated that the end effector 88 may be attached to the transmission rod 86 by any suitable means, such as a welded joint or the like. Although the end effector 88 may be detachable from the transmission rod 86, it is also contemplated that the end effector 88 and transmission rod 86 may be formed as a single unit.

The end effector 88 may have a distal region 88b having a smaller cross-section area than a proximal region 88a thereof, thereby forming a vibrational amplitude step-up junction. The step-up junction acts as velocity transformer as known in the art, increasing the magnitude of the ultrasonic vibration transmitted from the proximal region 88a to the distal region 88b of the end effector 88.

The end effector 88 preferably has a length substantially equal to an integral multiple of one-half system wavelengths (nλ/2). The end effector 88 is disposed at an antinode in order to produce the maximum longitudinal deflection of the distal end. When the transducer assembly 82 is energized, the distal end of the end effector 88 is configured to move longitudinally in the range of, for example, approximately 10 to 500 microns peak-to-peak, and preferably in the range of about 30 to 100 microns at a predetermined vibrational frequency, and most preferably at about 90 microns.

The end effector 88 is preferably made from a solid core shaft constructed of material which propagates ultrasonic energy, such as a titanium alloy (i.e., Ti-6Al-4V) or an aluminum alloy. The end effector 88 may be fabricated from other suitable materials. It is also contemplated that the end effector 88 may have a surface treatment to improve the delivery of energy and desired tissue effect. For example, the end effector 88 may be micro-finished, coated, plated, etched, grit-blasted, roughened or scored to enhance coagulation in tissue. Additionally, the end effector 88 may be sharpened or shaped to enhance its energy transmission characteristics. For example, the end effector 88 may be blade shaped, hook shaped, or ball shaped.

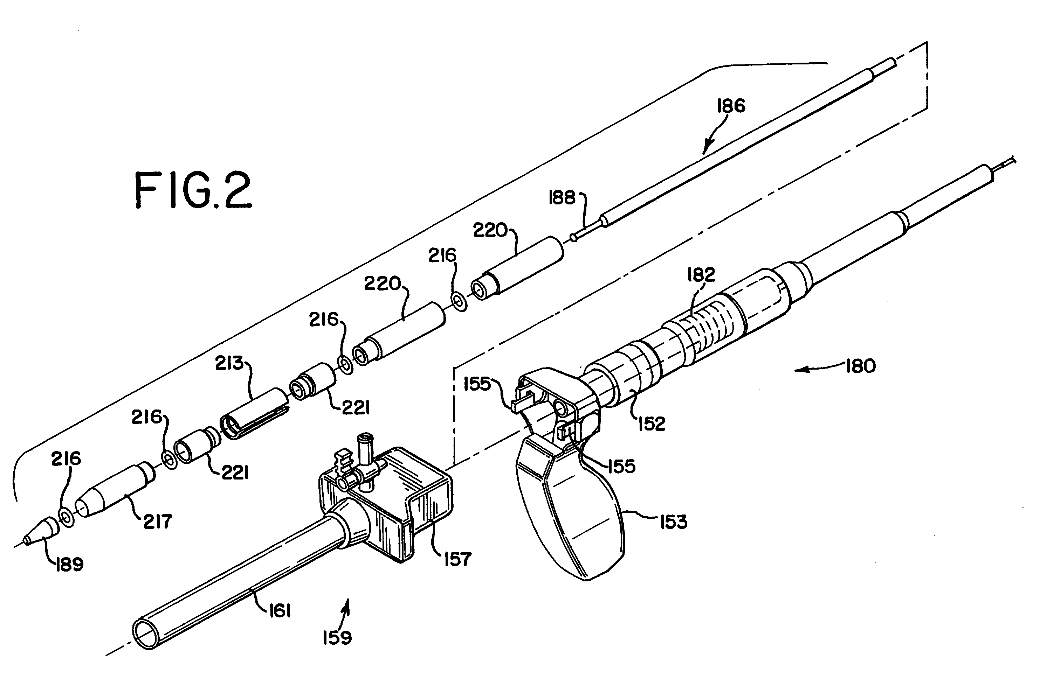

With particular reference now to FIGURE 2, therein is illustrated the ultrasonic trocar assembly embodying the principles of the present invention. The present trocar assembly includes an acoustic assembly configured as an obturator 180, which in many respects corresponds in construction and function to the previously described acoustic assembly of surgical system 10. Components of the present trocar assembly which generally correspond to those components of surgical system 10 are designated by like reference numerals in the one-hundred and two-hundred series.

The obturator 180 of the present trocar assembly includes a housing 152 having positioned therein a transducer assembly 182 configured in accordance with previously-described transducer assembly 82. To facilitate manipulation of the obturator 180, a handgrip 153 is preferably provided which is joined to the housing of the device. A pair of manually operable locking clips 155 are preferably provided on the upper portion of the handgrip 153 for cooperative releasable and locking engagement with a proximal housing 157 of the trocar cannula 159 of the present trocar assembly. The trocar cannula 159, including a tubular cannula 161, is positionable in releasable locking engagement with the obturator 180 of the present assembly, with the obturator 180 arranged in generally telescopic relationship coaxially within the tubular cannula 161.

As noted, the obturator 180 is configured generally in accordance with the previously described acoustic assembly 80 of surgical system 10. To this end, the obturator 180 includes a generally elongated waveguide 186 having a distal end effector portion 188. The end effector 188 can be unitary (i.e., one-piece) with the remaining length of the waveguide, or the end effector can be a separate component joined to the elongated waveguide such as by a threaded connection or the like. The waveguide 186 is operatively coupled to the transducer assembly 182 by a suitable mounting element 184, whereby ultrasonic energy is transmitted through the waveguide from the transducer assembly.

In accordance with the present invention, a generally frusto-conical sheath-like interface member 189 is provided so that it generally surrounds the end effector 188 of waveguide 186. The interface member 189 is non-vibratory, that is, substantially isolated from the ultrasonic energy directed through the waveguide 186 from the transducer assembly positioned within housing 152.

In the preferred form, elongated waveguide 186 is housed within a generally tubular sheath-like non-vibratory housing positioned generally coaxially about the waveguide 186. In the illustrated embodiment, this preferred arrangement is provided in the form of a plurality of tubular sheath members 220 which carry therein a plurality of compliant isolation supports 216 which function to desirably isolate the tubular sheath members from the ultrasonic energy carried by waveguide 186. The supports 216 thus function in the nature of the supports 116 of the acoustic assembly 80 of previously-described surgical system 10. Tubular members 220 may be assembled by snap-fitment, threaded connection, or other suitable means.

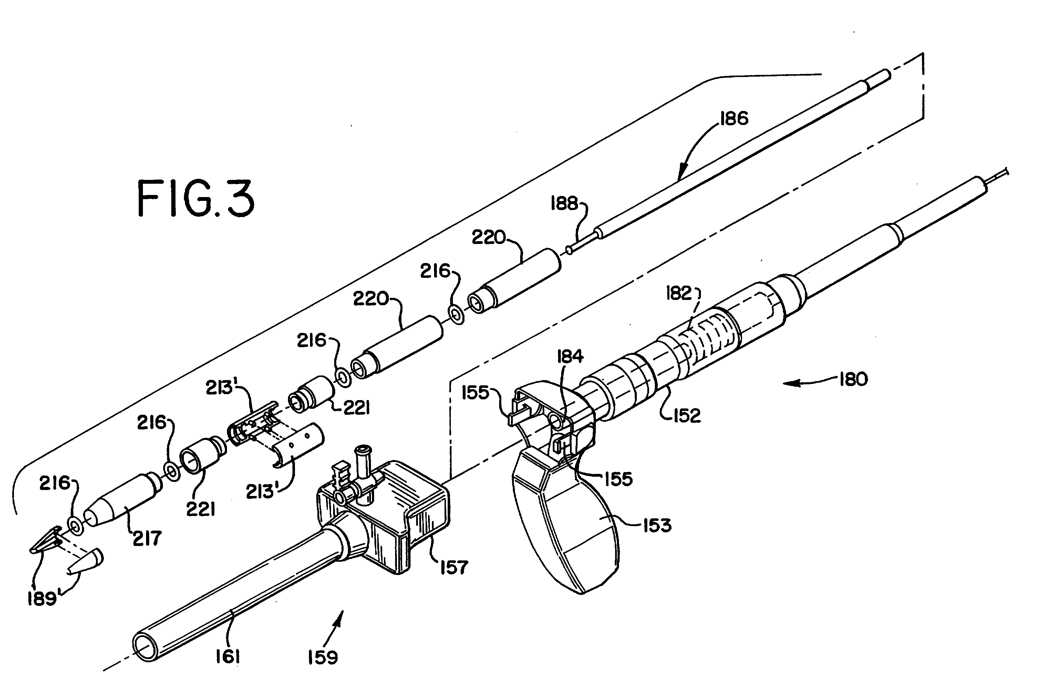

In each of the illustrated embodiments of the present trocar assembly, assembly of the outer sheath-like housing of the distal end portion of the obturator 180 is facilitated by the provision of a pair of sheath portions 221 which fit in cooperating relationship with a coupling member 213. The coupling member 213 illustrated in FIGURE 2 as a slit sleeve, and alternately illustrated in the embodiment of FIGURE 3 as a pair of split sleeves 213', cooperatively receives the sheath portions 221, while permitting the distal-most sheath portion 221 to be removed from the coupling 213, 213' as may be required.

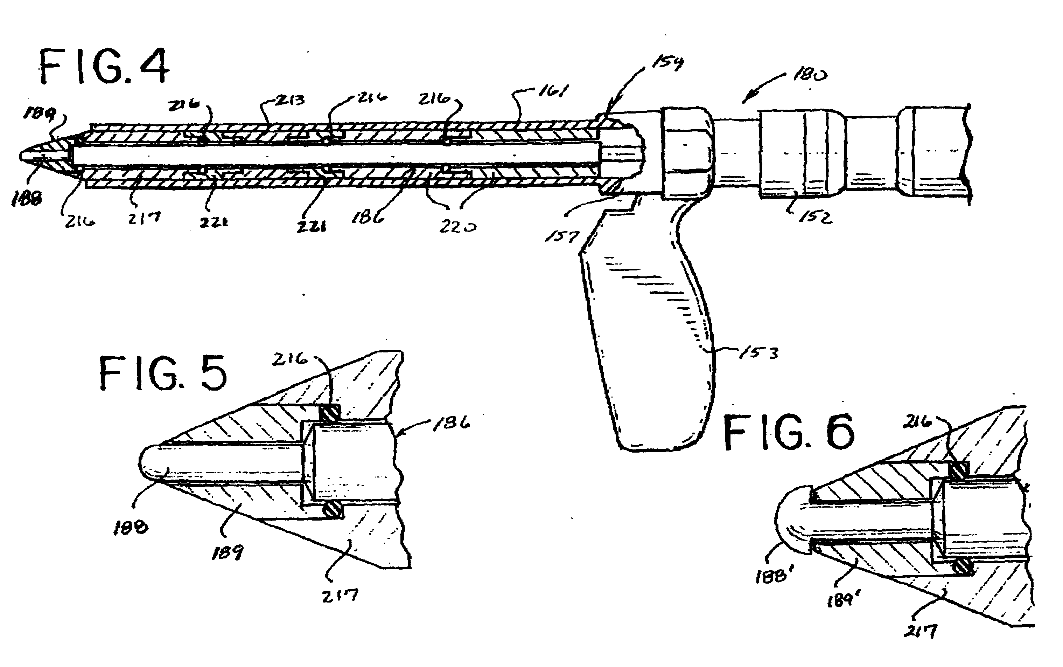

The outer tubular housing of the obturator 180 further includes an interface body 217 which is operatively connected with the distal-most sheath portion 221, again with an isolation support 216 preferably provided for isolating the interface body 217 from the vibratory energy of the internally-positioned waveguide 186. The interface member 189 is positionable generally within the free end portion of the interface body 217, as illustrated in detail in FIGURE 5, the preferably frusto-conical outer surface of the interface member 189 positioned to merge into the preferably frusto-conical outer distal surface of interface body 217.

While it is contemplated that the interface member 189 may be unitary, it is within the purview of the present invention to provide the interface member in a two-piece configuration, such as illustrated in FIGURE 3 at 189'. When the interface member is configured to comprise a pair of half portions 189' as illustrated in FIGURE 3, the half portions 189' can be provided so as to together define an inside diameter less than an outside diameter of the associated end effector 188', as shown in FIGURE 6. The interface member can thus be "recessed" to below the "profile" of the end effector, and have an outside diameter at its distal end less than the outside diameter of the end effector. While the interface member can be formed from a wide variety of materials, the member preferably comprises Ultem®, or other suitable polymeric or metallic material.

As illustrated in FIGURE 5, interface member 189 comprises a distal portion spaced from the free end of the end effector 188 to thereby define an exposed end portion for the end effector. By the illustrated generally frusto-conical configuration of the interface member, the interface member surrounds the end effector 188, with the outwardly, rearwardly diverging configuration of the interface member providing the desired enlargement and dilation of a penetration opening initially formed ultrasonically by the end effector 188.

In a preferred embodiment, the exposed end portion of the end effector has a rounded, blunt (i.e., non-sharpened) configuration, and preferably has a diameter of about 1.0 mm to about 3.0 mm, most preferably about 2.5 mm. The end effector is preferably configured such that substantially only a forwardly facing surface of the end effector effects penetration of body tissue to create the penetration opening. The desired dilation and enlargement of a penetration opening formed by the end effector is facilitated by configuring the interface member to have an outward taper at an angle of about 10° to about 60° from the centerline of the waveguide 186. It is contemplated that the ratio of the outside diameter of the free end of the end effector 188 (and 188') to the outside diameter of the interface member 189 (and 189') is about 1:5 to about 1:2 (i.e., the end effector has an outside diameter which is about 20% to about 50% of the outside diameter of the interface member).

The interface member 189 may be fitted with the interface body 217 by a press-fit, a suitable threaded connection, adhesive bonding, fusing, or other suitable connection arrangement. While the waveguide 186 and its end effector 188 transmit ultrasonic energy through the system, it is contemplated that the interface member 189 and interface body 217, as well as the components of the sheath-like tubular housing fitted about the waveguide, be non-vibratory and isolated (by isolation supports 216) from the vibratory energy being transmitted through the waveguide.

From the foregoing description, use of the trocar assembly of the present invention will be readily appreciated. The ultrasonic obturator 180 of the assembly is positioned telescopically within the tubular cannula 161 of the trocar cannula 159. Locking clips 155 are preferably engaged with the proximal housing 157 of the trocar cannula 159, thus permitting the entire instrument to be readily manipulated as a unit by handgrip 153. With the obturator positioned within the cannula, the free end of the obturator, including end effector 188 and interface member 189 project from the distal end portion of the tubular cannula 161.

The trocar assembly is next positioned so that the free end portion of the ultrasonic obturator is positioned in contact with the wall of the body cavity to be penetrated. Either prior to or subsequent to such positioning, the ultrasonic system is energized, thereby effecting transmission of ultrasonic energy from the transducer assembly within housing 152 through the waveguide mount 184, and through the waveguide and end effector. By application of suitable pressure via the handgrip 153, the end effector 188 can be caused to penetrate the wall of the body cavity as the ultrasonic energy being transmitted through the waveguide acts on the cellular structure of the wall.

As noted, it is contemplated that the exposed end portion of the end effector 188 be provided with a diameter of about 1.0 mm to about 3.0 mm., preferably about 2.5 mm, with working tip displacement or amplitude being on the order of about 30 to about 120 microns. Attendant to ultrasonic formation of a penetration opening in the wall of the body cavity, the interface member 189 acts as a sheath or cap about the end effector. Hemostasis of the penetration opening is desirably aided by the ultrasonic cutting effect.

After formation of the penetration opening by the ultrasonic cutting of end effector 188, operation of the ultrasonic system can be terminated. The obturator 180 is advanced distally so that the rearwardly diverging interface member 189, and the associated interface body 217, cooperate with the initiallyformed penetration opening to dilate and enlarge the opening, while minimizing patient trauma. As the obturator 180 is advanced distally, the initially-formed penetration opening becomes sufficiently enlarged as to permit subsequent introduction of the tubular cannula 161 of the trocar cannula 159. The cannula is then advanced through the dilated and enlarged opening, with the ultrasonic obturator 180 removed prior or subsequent to introduction of the tubular cannula 160 through the enlarged penetration opening. Operation of locking clips 155 permits the obturator 180 to be detached and completely removed from the trocar cannula 159, and the desired endoscopic surgical procedure performed. Because of the radial dilation effected during advancement of the cannula, subsequent to ultrasonic penetration, the so-called pull-out force necessary to remove the tubular cannula is desirably increased, thereby acting to maintain the cannula in position during the endoscopic procedure.

It is contemplated that use of the present invention by which a relatively small penetration opening is ultrasonically formed will further facilitate endoscopic procedures, in that the opening remaining after the completion of the procedure will be small enough as to preclude the need for surgical closing of the opening. It is also contemplated that use of the present invention will facilitate cost effective procedures, in that the ultrasonic obturator can be reused (within the limits of sterilization cycles) with the trocar cannula being the only disposable component of the system. It is contemplated that the invention can be embodied to employ a standard trocar cannula so this component can be disposed of at low cost.

It is preferred that the interface member (189, 189') which effects tissue dilation be removable for use of differently sized interface members with differently sized trocar assemblies (e.g., 5 mm, 7/8 mm, 10/11 mm, 11/12 mm). While the interface member may be frusto-conical, the member can be otherwise shaped, such as in the form of an outwardly curving "fish tail", to "engineer" a specific dilation.

It is within the purview of the present invention that a feedback system be provided, such as disclosed in U.S. Patent No. 5,449,370, for adjusting ultrasonic energy levels in response to variations in load. Although not illustrated, it will be appreciated that the present invention can be configured to include any of a variety of heretofore known mechanical mechanisms which can be employed for controlling penetration of the obturator, including springs, latches, shields, and the like.

From the foregoing, it will be observed that numerous modifications and variations can be effected without departing from the true spirit and scope of the novel concept of the present invention. It is to be understood that the present disclosure is intended as an exemplification of the invention, and is not intended to limit the invention to the specific embodiment illustrated. The disclosure is intended to cover, by the appended claims, all such modifications as fall within the scope of the claims.

## Claims

1. An ultrasonic trocar obturator for use with an associated trocar cannula, comprising:
a housing;
an ultrasonic transducer assembly positioned within said housing;
an ultrasonic waveguide extending from said housing, said waveguide including a proximal end operatively coupled to said transducer assembly for transmission of ultrasonic energy through said waveguide, said waveguide further including an end effector at the distal end thereof; and
an interface member surrounding and diverging outwardly of the end effector of said waveguide, so that substantially only a forwardly facing surface of said end effector effects penetration of body tissues and creates a penetration opening, with said interface member thereafter dilating the penetration opening by distal movement of said obturator to facilitate insertion of the associated trocar cannula.

2. An ultrasonic obturator in accordance with claim 1, including
a tubular sheath surrounding said ultrasonic waveguide and extending from said housing to said interface member.

3. An ultrasonic obturator in accordance with claim 2, including
at least one isolation element positioned between said tubular sheath and said ultrasonic waveguide for isolating said tubular sheath from the ultrasonic waveguide.

4. An ultrasonic obturator in accordance with claim 1, wherein
said interface member comprises a distal portion spaced from the free end of said end effector to define an exposed end portion thereof providing said forwardly facing surface, said distal portion of said interface member having an outwardly, rearwardly diverging configuration for effecting dilation of said penetration opening, wherein the ratio of an outside diameter of said free end of said end effector to an outside diameter of said interface member is about 1:5 to about 1:2.

5. An ultrasonic obturator in accordance with claim 4, wherein
said exposed end portion of said end effector has a diameter of about 2.5mm.

6. An ultrasonic obturator in accordance with claim 4, wherein
said distal portion of said interface member tapers outwardly at an angle of about 10° to about 60°.

7. An ultrasonic obturator in accordance with claim 1, wherein
said interface member comprises a pair of half portions which together define an inside diameter less than an outside diameter of said end effector.

8. A method of inserting a trocar cannula into a body cavity, comprising the steps of:
providing an ultrasonic trocar obturator having an ultrasonic waveguide including an end effector at a distal end thereof, and an interface member surrounding and diverging outwardly of said end effector wherein the ratio of an outside diameter of a free end of said end effector to an outside diameter of said interface member is about 1:5 to 1:2;
positioning said trocar obturator within a trocar cannula;
positioning said trocar obturator in.contact with a wall of said body cavity, and ultrasonic vibrating said waveguide so that said end effector creates a penetration opening;
dilating said penetration opening with said interface member by distally advancing said ultrasonic obturator into said penetration opening; and
reinserting said trocar cannula into said body cavity by advancing said trocar cannula into the dilated penetration opening.

9. A method of inserting a trocar cannula in accordance with claim 8, including
removing said ultrasonic trocar obturator from within the trocar cannula after insertion of said cannula into said dilated penetration opening.

10. A method of inserting a trocar cannula in accordance with claim 8, wherein
said step of providing said ultrasonic trocar obturator includes configuring said interface member to have an outside diameter at its distal end less than an outside diameter of said end effector.
